# EUROPEAN PATENT APPLICATION

(11) **EP 4 838 222 A1**
(43) Date of publication of application: **11.11.2026**
(21) Application number: 26177074.7
(22) Date of filing: 07.05.2026
(51) Int. Cl.: G01N 33/533, A61K 49/00, G01N 33/58

(54) **SITE-SPECIFIC BIOCONJUGATION OF FLUORESCENT SCAFFOLDS TO PROTEINS**

(30) Priority: 08.05.2025 EP 25175024
(71) Applicant: Miltenyi Biotec B.V. & Co. KG, 51429 Bergisch Gladbach (DE)
(72) Inventor: Zavoiura, Oleksandr, 51429 Bergisch Gladbach (DE); Yushchenko, Dmytro, 51429 Bergisch Gladbach (DE); Volkmann, Achim, 51429 Bergisch Gladbach (DE); DOSE, Christian, 51429 Bergisch Gladbach (DE); WIECZOREK, Marek, 51429 Bergisch Gladbach (DE)
(74) Representative: Kisters, Michael Marcus

(57) **Abstract**

The invention is directed to a method for providing a full length antibody having at least one Sortase recognition sequence at each heavy chain C-terminus with at least one fluorescent dye via DBCO click chemistry, the thus obtained conjugate and a method for using the conjugate for reversable detecting a target moiety in a sample of biological specimens.

## Description

The present invention is directed to site-specific bioconjugation of fluorescent scaffolds to proteins; and application thereof in cell analysis of biological specimens.

### BACKGROUND

Antibodies modified with fluorescent scaffolds are effective tools in the field of medical diagnostics, providing unique insights into pathological states. Fluorescent scaffolds (e.g. fluorescent proteins, polysaccharides, synthetic polymers, etc.) are bigger and brighter labels than small molecule organic dyes, thereby enabling higher sensitivity of detection in cell analysis applications. Importantly, cleavable fluorescent polymers (such as dextran) enable the imaging of multiple targets on the same biological specimen, as was shown in US 10,197,561 B2. This is achieved by first staining the sample with the antibody conjugate, and subsequent enzymatic cleavage of bonds that connect the fluorescent moiety to the antibody.

As described in US 10,197,561 B2, fluorescent scaffolds can be conjugated to antibodies by chemical modification of naturally occurring amino acid residues: lysines or cysteines. While effective for some antibodies, this approach has limitations. First, it yields a heterogeneous mixture of antibody conjugates with poor control over the composition of the final product. Second, chemical modification of native amino acids can compromise antibody function, leading to performance failure in cell analysis applications.

An antibody contains >40 solvent accessible lysine residues. Chemical modification of lysines can only yield a heterogeneous mixture of products with varied degree of labeling, making it impossible to conjugate fluorescent scaffolds site-specifically. Furthermore, modification of lysine residues in the complementarity determining region of antibody will compromise its ability to recognize the target epitope.

In their native state, solvent exposed cysteine residues of antibody are not available for bioconjugation: they exist in oxidized form, forming interchain disulfide bonds. To enable bioconjugation, disulfides must first be reduced (e.g. using TCEP or DTT) to yield a reactive thiol group, which can then react with an electrophile. The use of reducing agents can lead to antibody denaturation and decreased performance, or even complete functionality loss. This renders reduction chemistry incompatible with antibodies containing ≥8 solvent exposed cysteines, and cysteine-rich proteins in general.

Sortase-mediated reaction between a nanobody and an N-terminal triple Glycine of a peptide was described in US 2018/0280551A1. Described peptides were generated via solid phase peptide synthesis, and contained a triple Glycine residue for enzymatic reaction.

These peptides contained a single label for downstream application (such as radiologic imaging). In some versions, such radiolabeled and fluorescently nanobodies were modified with a second nanobody to generate bivalent constructs. While effective in radiologic imaging, these conjugates contain only one label (a radiolabel or a dye label), cannot be used for sequential imaging applications due to the lack of cleavable bonds, and require the use of synthetic peptides for enzymatic coupling.

### SHORT SUMMARY

We developed a method for site-specific attachment of fluorescent amino dextran to C-terminus of full length antibody; and use thereof for multiplexed imaging of biological specimens. The use of fluorescent dextran decorated with six fluorescent dyes provides high photon output in cell analysis applications and overcomes the limitations of fluorescent peptides with only one fluorescent reporter. Antibody conjugates described herein enable 870% higher sensitivity of detection in imaging application, compared to an antibody conjugate synthesized by chemical modification of its native cysteine residues. Finally, fluorescence signal of these conjugates can be erased by enzymatic cleavage of the bonds that connect the dyes to the antibody.

### OBJECT OF THE INVENTION

Object of the invention is a conjugate having the general formula (I) 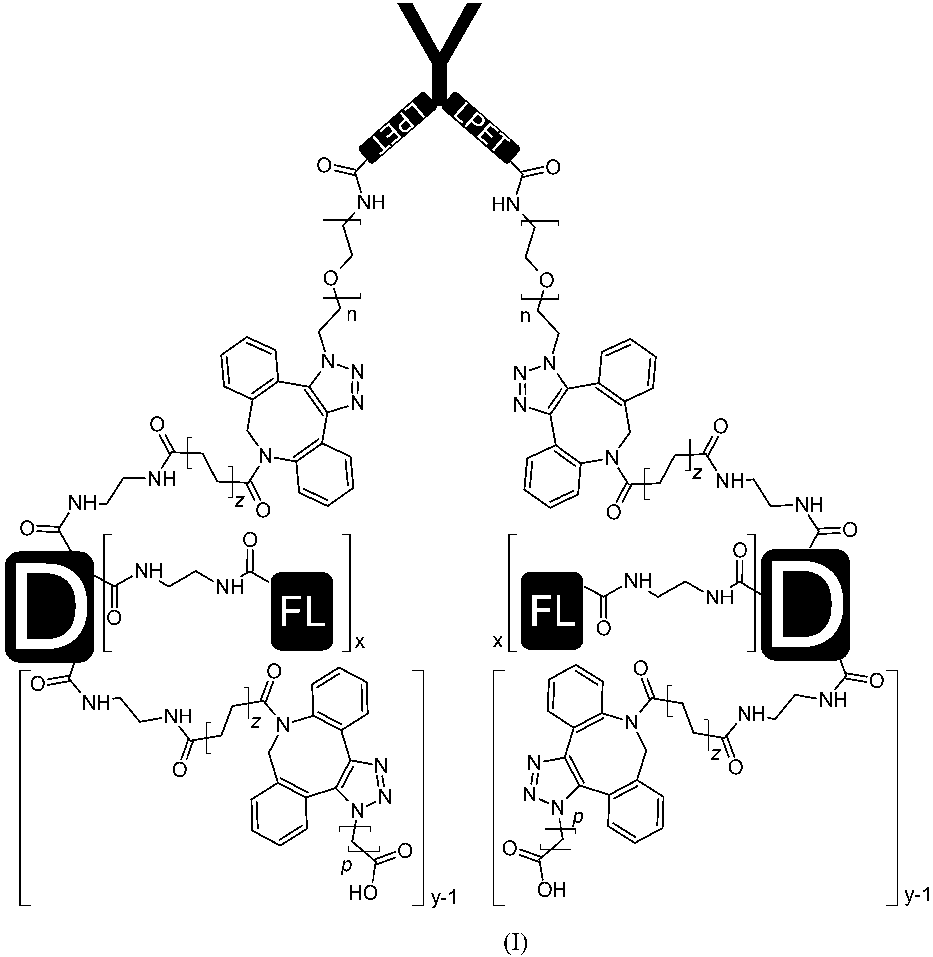 With
Y: full length antibody having the sequences LPET at each heavy chain C-terminus
D: polysaccharide having a molecular weight of 5-500 kDa, preferable 20-100 kDa
n: 1 to 3
z: 1 to 3
x: 1 to 40
y: 1 to 40
p: 1 to 20
FL: fluorescent dye

Another object of the invention is a method for providing a full length antibody having at least one Sortase recognition sequence at each heavy chain C-terminus with at least one fluorescent dye **characterized in**
a) providing the full length antibody with Sortase and a linker molecule having the general formula (II)

   H₂N-(CH₂-CH₂-O)ₙ-CH₂-CH₂-N₃ (II)

   with n = 1-3
   thereby obtaining a linker-antibody conjugate
b) providing the linker-antibody conjugate with a fluorescent reagent having the general formula III or IV 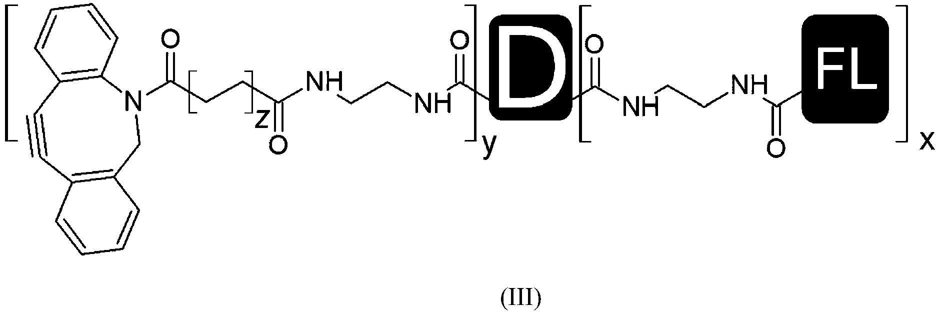 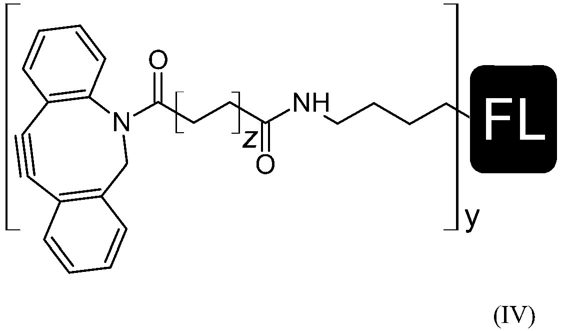
   with FL: fluorescent dye
   D: molecule polysaccharide having a molecular weight of 5-500 kDa
   y, x: independent 1 to 40
   z: 1 to 3
thereby reacting the N₃ groups of the linker-antibody conjugate with the DBCO group of the fluorescent reagent.

### BRIEF DESCRIPTION OF THE DRAWINGS

Fig. 1: Sortase-mediated reaction between an aminoethyl group of ethylene glycole derivative and the full length antibody comprising a sortase recognition motif.
Fig. 2: Synthesis of fluorescently modified amino dextran, and its modification with DBCO groups. Y = 4; X = 6.
Fig 3: Site-specific bioconjugation of fluorescent amino dextran to the C-termini of a full length antibody. X = 6.
Fig. 4: Fluorescence imaging of CD79b marker on FFPE-fixed human tonsil tissue with A) staining of CD79b positive cells and B) enhancement of detection sensitivity
Fig. 5: Schematic principle of dextranase-mediated signal erasure for multiplex imaging of multiple markers on the same tissue. X = 6.
Fig. 6: Fluorescence microscopy data showing that the fluorescence signal is removed from CD79b positive cells.
Fig. 7: Site-specific bioconjugation of fluorescent label, phycoerythrin, to the C-terminus of a full length antibody.
Fig. 8: Analytical SEC chromatograms confirm conjugation of fluorescent label, phycoerythrin, to the C-terminus of a full length antibody.

### DETAILED DESCRIPTION

The conjugate of the invention may comprise any full length antibody Y, for example as selected from the group consisting of anti-CD79b; anti-CD3; anti-CD4; anti-CD8; anti-CD11; anti-CD19; anti-CD39; anti-CD68; anti-CD163; anti-CD324; anti-CD326; anti-Ki67; anti-Cytokeratin; anti-mTOR; anti-IDOl; anti-Mamaglobin.

Due the preferred method of production, the full length antibody Y comprises the sequence "LPET" at both heavy chain C Termini.

Preferable, the fluorescent dye is selected from the group consisting cyanine, rhodamine, squaraine, BODIPY, fluorescein, phycoerythrin, allophycocyanin, and coumarin.

In the method for manufacturing the conjugate, it is possible to use fluorescent reagents with the general formula (III) or (IV) 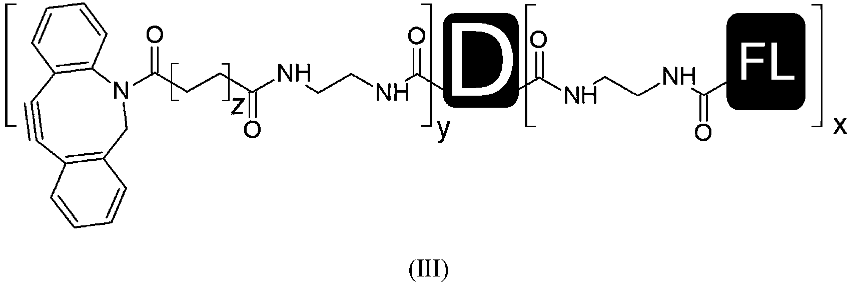 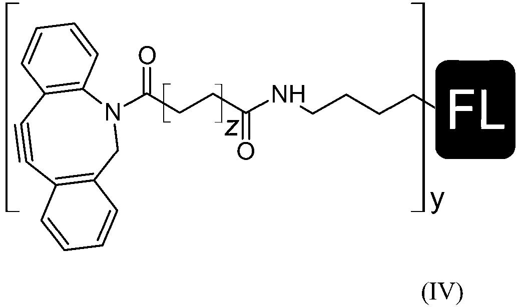
with FL: fluorescent dye
D: molecule polysaccharide having a molecular weight of 5-500 kDa
y, x: independent 1 to 40
z: 1 to 3

If the fluorescence reagent according to formula (IV) is used, the final conjugate does not contain a dextran unit.

In any case, it is preferred to use the sequence LPETG or LPETGG as Sortase recognition sequence in the full length antibody. The sortase enzyme recognizes this sequence and mediates the addition of the linker molecule with general formula (II). During the mediation, the Sortase recognition sequence is reduced to LPET, i.e. the final conjugate comprises this sequence.

Preferable, the polysaccharide is amino dextran.

In a embodiment of the method according to the invention, the unreacted DBCO groups can be deactivated using an azido alkanoic acid.

In another embodiment of the method according to the invention, in step b, the linker-antibody conjugate with a fluorescent reagent having the general formula (III) is provided in an amount (i.e. ratio) that the compound according to general formula (V) is obtained (where all indces have the maening as already disclosed)

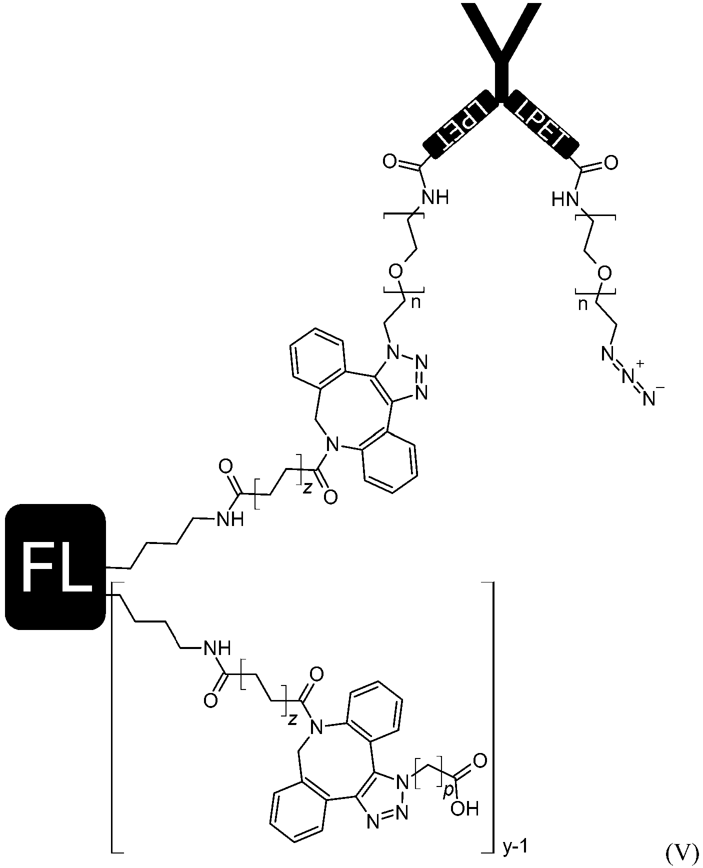

### USE OF THE INVENTION

The conjugate according to the invention with formula (I) is especially useful in a method for reversable detecting a target moiety in a sample of biological specimens by
a) providing at least one conjugate according to general formula (I) to the sample
b) labelling the target moiety recognized by the full length antibody Y with at least one conjugate
c) detecting the labelled target moiety via the fluorescent dyes FL
d) removing the fluorescent dyes FL by providing an enzyme capable of digesting the polysaccharide D.

The technical details of such detecting process are known to a person skilled in the art, for example from EP3037821A1.

### EXAMPLES

We produced the derivative of sortase A, first described in US9267127B2, in *E.coli.* Our variant comprised two His-tags at the N-terminus to simplify the purification; full sequence of this Sortase A is shown below.

Briefly, 50 ml precultures in vLB-medium (10 g/L NaCl, 10 g/L peptone, 5 g/L yeast extract) were set up at 37 °C for 16-18 hours, shaking at 250 rpm, one day prior to production and used for inoculation of main cultures in vLB-medium with a final OD600 of 0.1. Main cultures were grown in 1 L vLB-medium under the same conditions and induced with 0.4 mM IPTG (Carl Roth) at an OD600 of 0.6 - 0.8. After 3 - 4 h cells were pelleted at 4000xg, 4 °C for 20 min.

The pellets were dissolved in 20-40 ml IMAC wash buffer (50 mM TRIS, pH 8, 50 mM NaCl, 5 mM Imidazole) and lysed by sonification (alternative: French press with 1200 bar). After lysis, the samples were centrifuged at 8.000xg for 20 min and the supernatant sterile-filtered through a 0.45 µm and 0.22 µm filter. Then, 2 mL Ni²⁺ sepharose beads (Cytiva) were loaded into a column, equilibrated with 10 CVs of washing buffer 1 (50 mM TRIS, 50 mM NaCl, pH 8, 5 mM imidazole) and the samples were loaded onto the column by gravity flow. Then, 40 mL washing buffer 1 was added for the first washing step, followed by 20 mL washing buffer 2 (50 mM TRIS, 50 mM NaCl, pH 8, 10 mM imidazole). The protein was eluted with 15 ml elution buffer (50 mM TRIS, 50 mM NaCl, pH 8, 350 mM imidazole).

Directly after IMAC-purification, the elution fraction was diluted with 50 mM MES buffer, pH 6.3 to a conductivity < 4 mS/cm. A 5 mL High Trap SP-sepharose column (Cytiva) was equilibrated with 10 CV 50 mM MES buffer, using a peristaltic pump with a flow rate of 5 mL/ min. The solution was loaded onto the column with a flow rate of 5 mL/ min. After loading, the column was washed with 50 mM MES buffer, pH 6.3 (10 CVs). The elution was carried out by applying a NaCl (50 mM MES buffer, pH 6.3, 1 M NaCl) gradient to the column over 10 CVs for 20 min with a flow rate of 2.5 ml/ min. Finally, the protein was buffer exchanged against 20 mM Tris, 50 mM NaCl, pH 8 using a HiPrep desalting column (cytiva HiPrep 26/10) according to the manufacturer's instruction.

| | |
|---|---|
| Sortase A sequence | MSHHHHHHAGSHHHHHHAGSQAKPQIPKDKSKVAGYIEIPDADIKEP VYPGPATREQLNRGVSFAEENESLDDQNISIAGHTFIDRPNYQFTNLK AAKKGSMVYFKVGNETRKYKMTSIRNVKPTAVGVLDEQKGKDKQL TLITCDDYNEETGVWETRKIFVATEVK. |

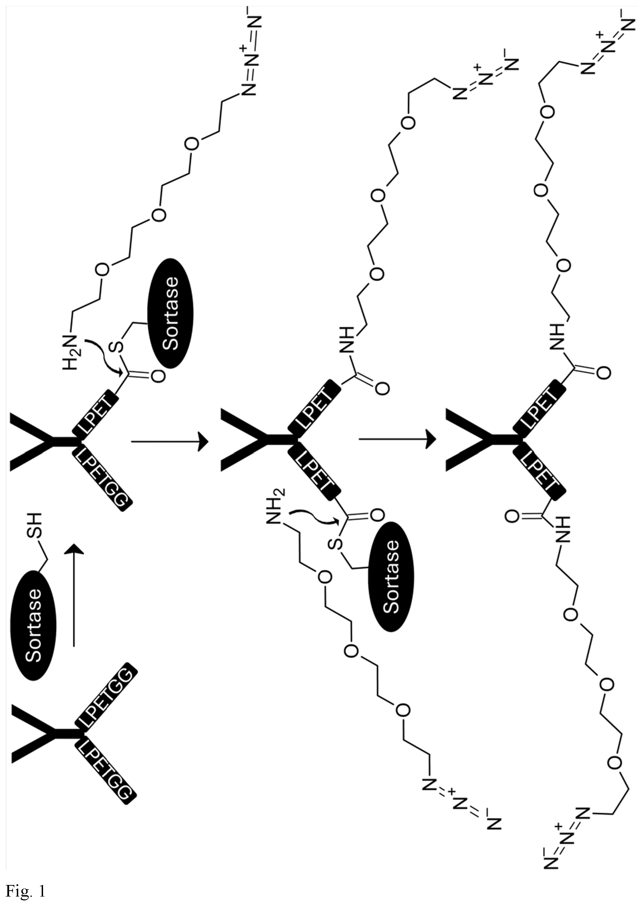

In order to synthezize the compound of the invention, a sortase-mediated reaction between an aminoethyl group of ethylene glycole derivative and the full length antibody comprising a sortase recognition motif is conducted according to Fig. 1. To synthesize the conjugate, an antibody was expressed with an LPETGG motif at each of its heavy chain C-termini. Then, the antibody was mixed with 100 equivalents of the linker (O-(2-Aminoethyl)-O'-(2-azidoethyl)diethylene glycol), 0.1 equivalents of sortase A and the mixture was incubated in TRIS buffer pH 7.8 for 22 h at 21 °C. Reaction was stopped by the addition of EDTA, and excess linker removed using Amicon centrifugal filter.

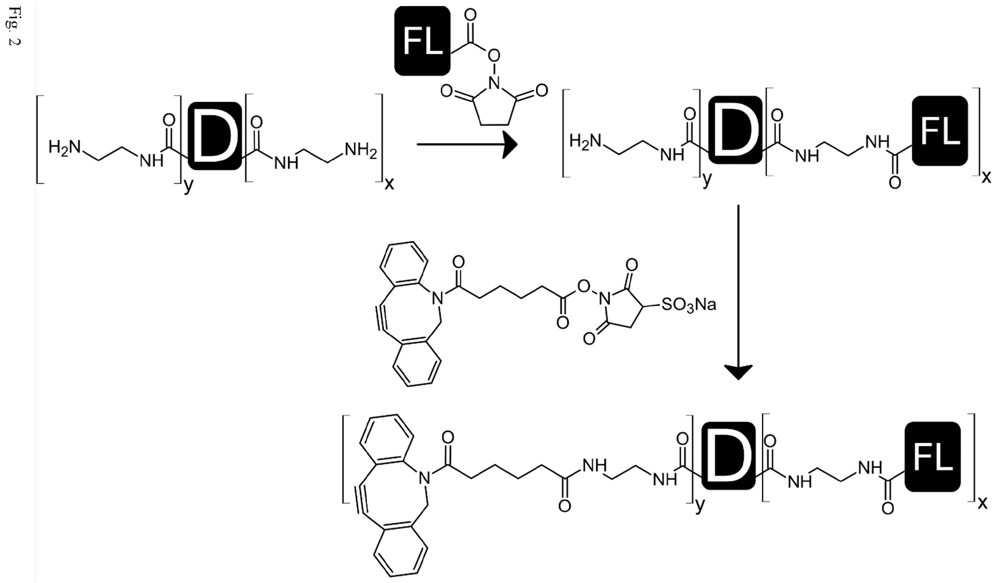

Figure 2. shows schematically the synthesis of fluorescently modified amino dextran, and its modification with DBCO groups. To synthesize this material, amino dextran (70 kDa, 10 amino groups, Fina biosolutions) was mixed with seven molar equivalents of NHS ester of Vio667^{®} in PBS/EDTA buffer spiked with 10% carbonate buffer. Reaction mixture was incubated at 21 °C for one hour. Then the product was purified via Amicon centrifugal filter from the remaining dye. In the second step, fluorescent dextran was incubated with twenty equivalents of DBCO-Sulfo-NHS ester (Broadpharm) in PBS/EDTA for one hour at 21 °C. Excess linker was removed using NAP5 column. Finally, the material was pre-concentrated in Amicon centrifugal filter. The number of DBCO groups on dextran was quantified using an indirect UV-Vis spectroscopy assay. To that end, Dextran-Vio667-DBCO was incubated with twenty molar equivalents of FAM-azide for 2 h and purified via Amicon centrifugal filter. Then, the absorbance at 494 nm was measured to quantify the number of reactive DBCO groups.

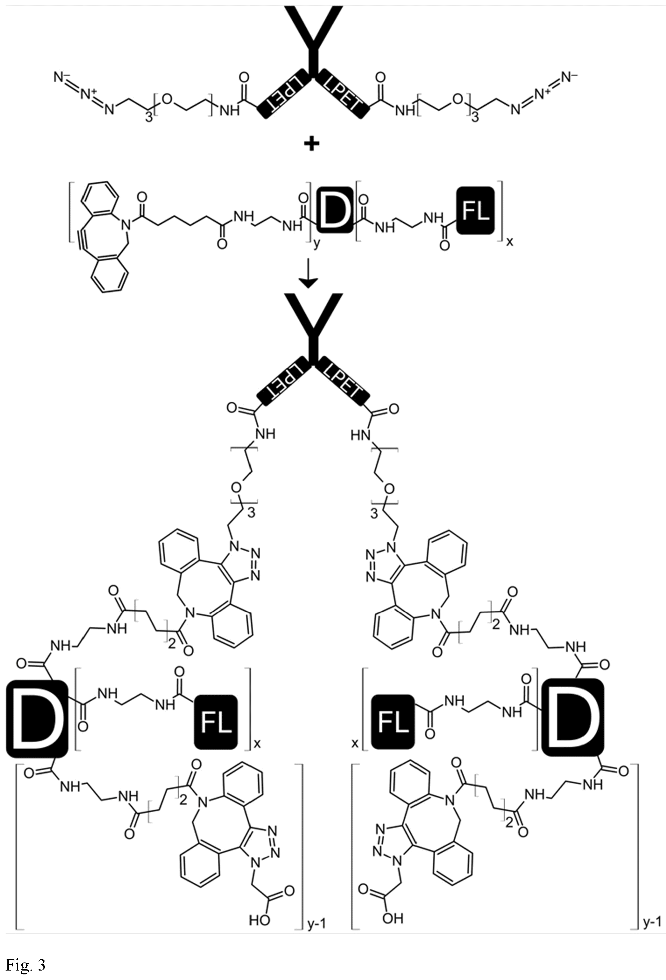

Figure 3 schematically shows site-specific bioconjugation of fluorescent amino dextran to the C-termini of the full length antibody. An antibody comprising a diethylene glycol and an azide group was incubated with two equivalents of fluorescent DBCO-modified dextran overnight (at room temperature). Reaction was stopped by the addition of 20 equivalents of 2-azidoacetic acid (overnight incubation at 2-8 °C). Finally, the conjugate was purified using size exclusion chromatography (Äkta system, Superdex 200 column, Increase 10/300).

This method yields a homogeneous product with highly controlled composition, whereby only antibody regions not responsible for biomarker recognition are chemically modified. In contrast, antibody regions responsible for biomarker recognition (Fabs) remain chemically intact.

In addition, herein described method allows for conjugation of fluorescent scaffolds to cysteine-rich antibodies without the use of reducing agents (TCEP, DTT). It ensures that proteins do not undergo denaturation during bioconjugation, thereby retaining their function in cell analysis applications. This is in contrast to modification of native cysteine residues of antibody, which requires the use of reducing agents.

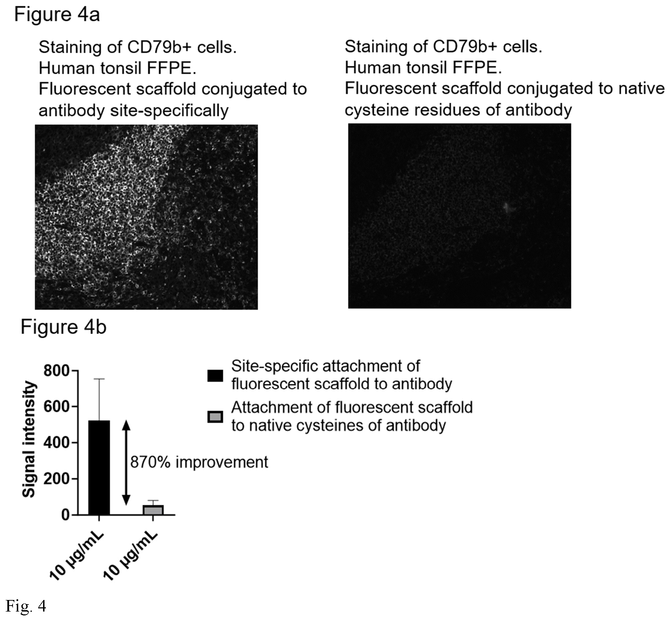

Figure 4 shows that site-specific attachment of fluorescent scaffold enhances detection sensitivity in fluorescence microscopy by 870%, compared to a nonspecific method of modifying native cysteine residues. In this experiment, FFPE fixed human tonsil samples were incubated with the respective anti-CD79b antibody conjugate for 10 min, then the samples were washed five times, and the images acquired. Co-staining with DAPI was performed to localize cell nuclei. Antibody conjugate with C-terminally attached fluorescent dextran detected CD79b epitopes with 870% higher sensitivity.

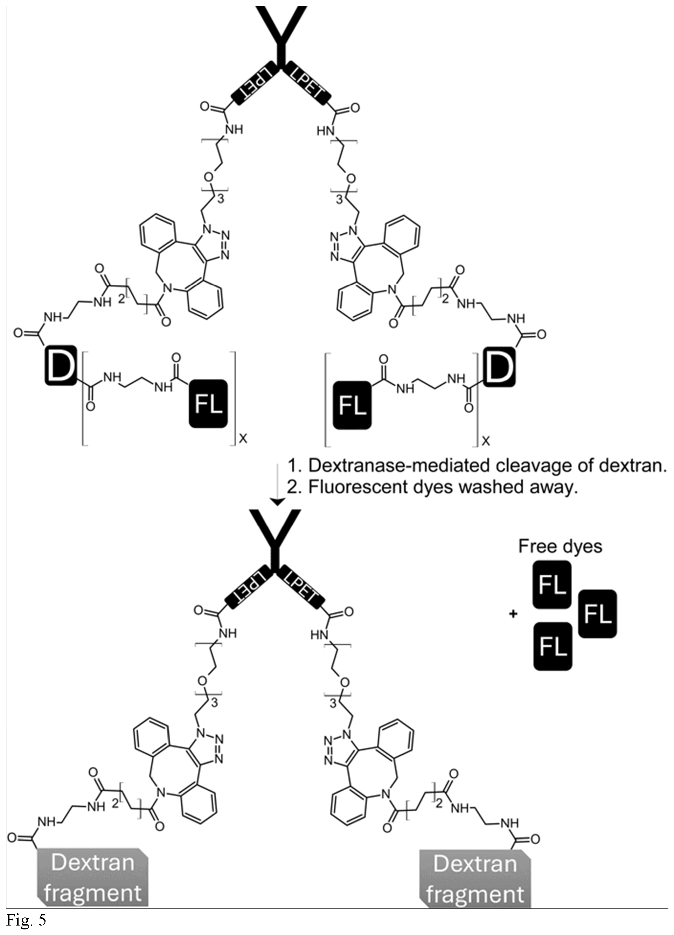

Figure 5 shows schematically the principle of dextranase-mediated signal erasure for multiplex imaging of multiple markers on the same tissue. Dextranase enzymatically cleaves glycosidic bonds that connect the dye to the antibody, then the dyes are washed away; as a result, fluorescent signal is removed. Afterwards, the same tissue sample can be used to image additional markers.

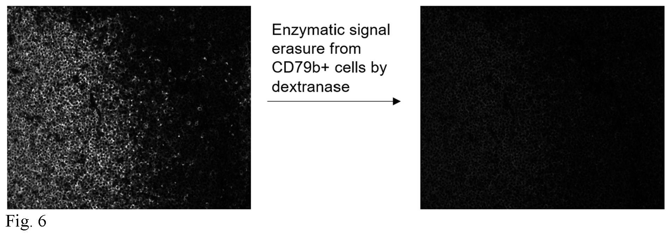

Figure 6 shows that antibody conjugate with site-specifically attached fluorescent dextran can be used for imaging, and then enzymatically degraded. Fluorescence microscopy data before and after dextranase treatment show that dextranase removes fluorescence signal from CD79b positive cells. This enables the reuse of the same tissue sample to image multiple markers, providing a unique multiplexing capability.

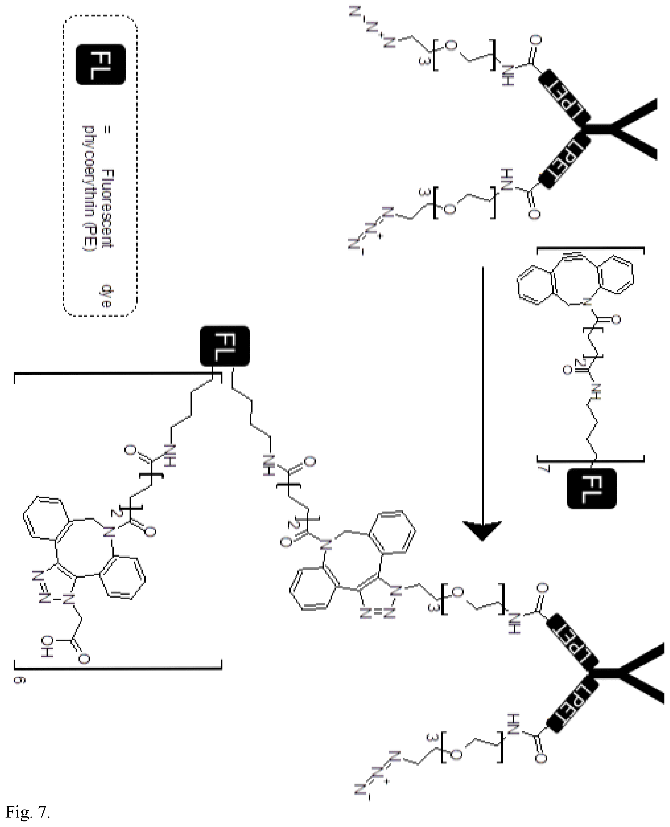

Figure 7 shows the synthesis of another compound of the invention - antibody conjugated to fluorescent label phycoerythrin (PE). In the first step (same mechanism as shown in Figure 1), antibody-azide intermediate was synthesized. For this, 10 mg of CD303 antibody comprising two LPETGG motifs were mixed with 10 mM of the linker (O-(2-Aminoethyl)-O'-(2-azidoethyl)diethylene glycol), and 0.4 equivalents of sortase in TRIS buffer, pH 7.8. Reaction mixture was incubated for 20 h at 20 °C. Afterward, the reaction was stopped by the addition of 125 µL of 0.5 M EDTA solution; excess linker was removed using Äkta FPLC System; Superdex 200 10/300 column was used. In the second step, PE was modified with DBCO. For that, 50 mg of PE were mixed with 20 equivalents of DBCO-Sulfo-NHS in 1xPBS with 5 mM EDTA buffer for 1 h at 20 °C. Unreacted linker was removed using a PD10 column. Finally, Antibody-azide intermediate was reacted with PE-DBCO intermediate. For this, 1.5 mg of antibody-azide were reacted with 3 molar equivalents of PE-DBCO in 1xPBS buffer with 5 mM EDTA buffer. The reaction was stopped by the addition of 100 molar equivalents of 2-azidoacetic acid (1 h, 20 °C). The resulting antibody-PE conjugate was purified on Äkta system using a Superdex 200 16/600 column. Purified sample was analyzed via analytical SEC chromatography, using Advanced Bio SEC column 300 Å; 2.7 µm; 4.6x300 mm from Agilent.

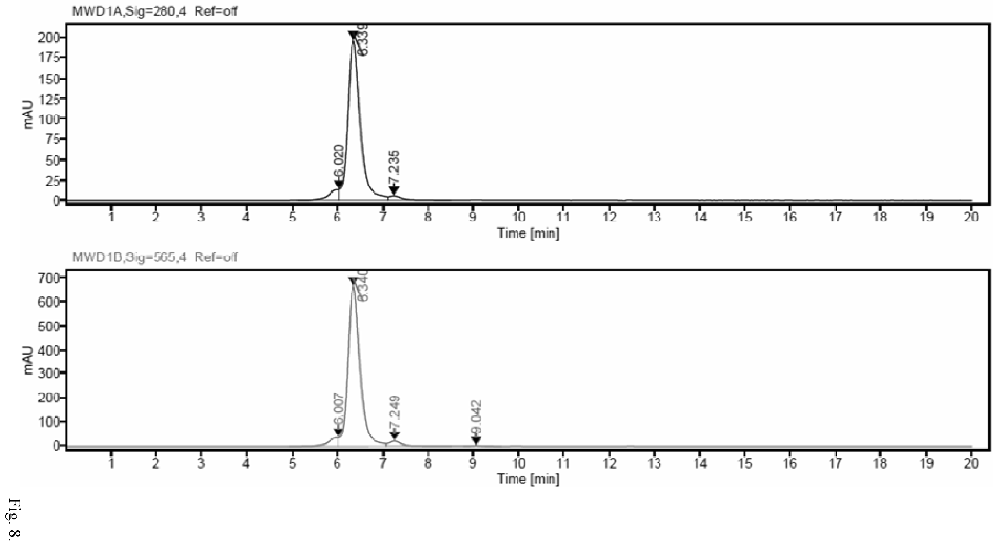

Figure 8 shows analytical SEC chromatogram of antibody-PE conjugate after purification; it confirms that phycoerythrin (PE) was conjugated to antibody.

## Claims

1. Conjugate having the general formula (I) 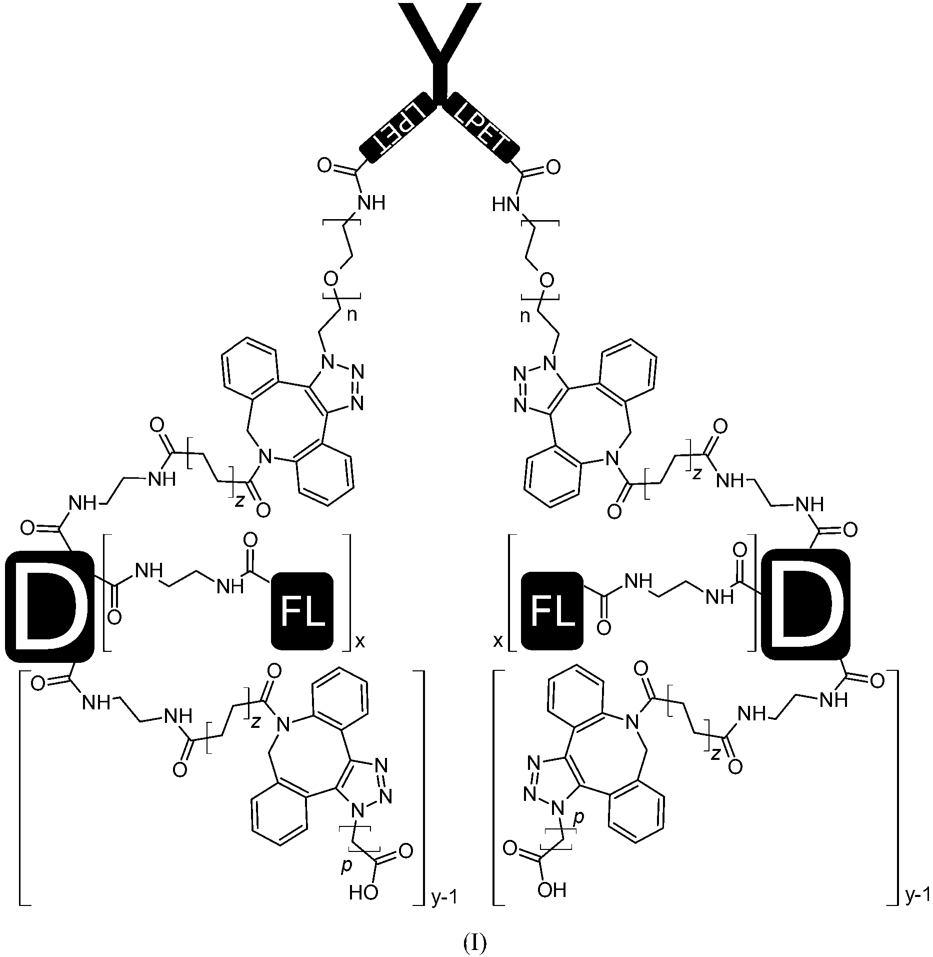 With
Y: full length antibody having the sequences LPET at each heavy chain C-terminus
D: polysaccharide having a molecular weight of 5-500 kDa
n : 1 to 3
x: 1 to 40
y: 1 to 40
z: 1 to 3
p: 1 to 20
FL: fluorescent dye

2. Conjugate according to claim 1 **characterized in that** the full length antibody Y is selected from the group consisting of anti-CD79b; anti-CD3; anti-CD4; anti-CD8; anti-CD11; anti-CD19; anti-CD39; anti-CD68; anti-CD163; anti-CD324; anti-CD326; anti-Ki67; anti-Cytokeratin; anti-mTOR; anti-IDO1; anti-Mammaglobin.

3. Conjugate according to claim 1 or 2 **characterized in that** the fluorescent dye is selected from the group consisting cyanine, rhodamine, squaraine, BODIPY, fluorescein, phycoerythrin, allophycocyanin, and coumarin.

4. Method for reversable detecting a target moiety in a sample of biological specimens by
a) providing at least one conjugate according to claim 1 to the sample
b) labelling the target moiety recognized by the full length antibody Y with at least one conjugate
c) detecting the labelled target moiety via the fluorescent dyes FL
d) removing the fluorescent dyes FL by providing an enzyme capable of digesting the polysaccharide D.

5. Method for providing a full length antibody having at least one Sortase recognition sequence at the heavy chain C-terminus with at least one fluorescent dye
**characterized in**
a) providing the full length antibody with Sortase and a linker molecule having the general formula (II)
H₂N-(CH₂-CH₂-O)ₙ-CH₂-CH₂-N₃ (II)
with n = 1-3
thereby obtaining a linker-antibody conjugate
b) providing the linker-antibody conjugate with a fluorescent reagent having the general formula (III) or (IV) 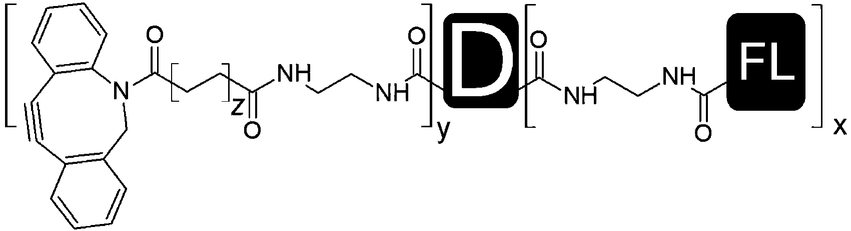 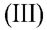 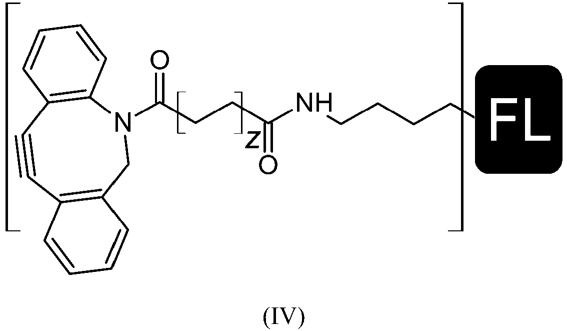
with FL: fluorescent dye
D: molecule polysaccharide having a molecular weight of 5-500 kDa y, x: independent 1 to 40
z: 1 to 3
thereby reacting the N₃ groups of the linker-antibody conjugate with the DBCO group of the fluorescent reagent.

6. Method according to claim 5 **characterized in that** the Sortase recognition sequence has the sequence LPETG or LPETGG.

7. Method according to claims 5 or 6 **characterized in that** the polysaccharide is amino dextran.

8. Method according to any of claims 5 to 7 **characterized in** deactivating the unreacted DBCO groups using an azido alkanoic acid.

9. Method according to any of claims 5 to 8 **characterized in that** in step b, the linker-antibody conjugate with a fluorescent reagent having the general formula (III) is provided in an amount that the compound according to general formula (V) is obtained
